# EUROPÄISCHE PATENTSCHRIFT

(11) **EP 2 492 663 B1**
(45) Veröffentlichungstag und Bekanntmachung des Hinweises auf die Patenterteilung: **04.12.2019**
(21) Anmeldenummer: 12155883.7
(22) Anmeldetag: 17.02.2012
(51) Int. Cl.: G01N 1/42, A01N 1/02, B65G 1/00, A61B 10/00, F25D 19/00, G01N 35/00, F25D 13/06

(54) **Kryolagersystem**
Cryo storage system
Système de stockage cryogénique

(30) Priorität: 28.02.2011 DE 102011012887
(43) Veröffentlichungstag der Anmeldung: 29.08.2012
(73) Patentinhaber: Askion GmbH, 07549 Gera (DE)
(72) Erfinder: Leuthold, Dirk, D-07554 Brahmenau (DE); Leuschner, Reiner, D-07549 Gera (DE); Schubert, Steffen, 07616 Serba (DE); Doms, Lutz, 08543 Pöhl (DE)
(74) Vertreter: Oehmke, Volker

(56) Entgegenhaltungen:
- WO-A2-2005/010499
- DE-T2- 69 230 405
- DE-U1-202007 002 712

## Beschreibung

Die Erfindung betrifft eine Kryolagersystem zur Aufnahme, Speicherung und Ausgabe von Proben, insbesondere von biologischen Proben sowie deren Handhabung, wie diese gattungsgemäß aus der DE 10 2008 057 981 B4 bekannt ist.

In Kryolagersysteme aufzubewahrende Proben, insbesondere biologische Proben, die üblicherweise in gepufferten Medien (z. B. Pufferlösungen) gelagert werden, sind zur Wahrung einer gleichbleibenden und reproduzierbaren Qualität bei Lagerbedingungen mit nur geringen Schwankungen zu speichern. Kritische Werte, beispielsweise zu hohe Temperaturen, sollen nicht oder nur sehr kurzzeitig erreicht oder gar überschritten werden. Solche Proben werden üblicherweise bei Temperaturen zwischen -20°C und -200°C gelagert.

Diese Anforderung an die Lagerbedingungen von Proben, und damit an die Kryolagersystem, sind besonders vor dem Hintergrund immer geringerer benötigter Probenmengen (z. B. einige Mikroliter für PCR- und/oder DNA-chip-basierte Verfahren) und einem damit verbundenen schnelleren Erwärmen und einem schnellen An- oder Auftauen der Proben von großer Bedeutung.

Kritische Werte können insbesondere dann erreicht werden, wenn die Proben zum Zwecke der Handhabung, z. B. zum Umsortieren und Kennzeichnen, zur Ergänzung oder Entnahme von Proben, aus einer Umgebung eines Lagers in eine Umgebung verbracht werden, in denen die Handhabungen erfolgen. Es ist üblich, eine Anzahl von Proben in Probenträgern (z. B. in sogenannten Racks) aufzubewahren. Soll lediglich eine Probe aus der Anzahl von Proben gehandhabt werden, sind aber trotzdem alle Proben des Probenträgers aus der Umgebung des Lagers zu verbringen, wodurch die Gefahr einer ungewollten Beeinträchtigung der Probenqualität gegeben ist.

In einer DE 10 2008 057 981 B4 ist eine Kryospeichereinrichtung zur Aufnahme, Speicherung und Ausgabe von Proben umfassend einen thermisch isolierten Kryotank (fortan Kryoraum) mit darin befindlichen Probenträgereinrichtungen (fortan: Probenträger) zur Aufnahme der Proben und einer verschließbaren Öffnung in einem oberen Deckenbereich des Kryoraums, über die der Kryoraum mit einem über dem Kryoraum angeordneten thermisch isolierten Zwischenlagerbehälter (fortan: Zwischenlager) in Verbindung steht; mit Transportmitteln zum Transport mindestens eines Probenträgers zwischen dem Kryoraum und dem Zwischenlager; mit einem seitlich an das Zwischenlager anschließenden Schleusenraum (fortan: Arbeitsraum), in dem eine Schleuse zur Einbringung von Proben aus einem Probentransportgefäß in den Arbeitsraum vorhanden ist und mit Mitteln zum Transport in mindestens einer horizontalen Richtung einzeln aus dem Probenträger entnehmbarer und wieder in diesen einsteckbarer Elemente eines im Zwischenlager befindlichen Probenträgers in den Arbeitsraum, beschrieben. Das Zwischenlager ist mittels einer Kühleinrichtung auf Temperaturen von -30°C bis -140°C kühlbar.

Die Transportmittel zum Transport der Probenträger zwischen Kryoraum und Zwischenlager sind als Winde, Roboterarm und/oder als ein ausfahrbares Gestänge ausgebildet.

Nachteilig an der Lösung der DE 10 2008 057 981 B4 ist, dass das Zwischenlager mehrere Probenträger aufnehmen kann und direkt in den Arbeitsraum übergeht. Durch die Wände des Zwischenlagers und des Arbeitsraums ist daher ein großes Volumen umschlossen, weshalb zur Gewährleistung einer ununterbrochenen Kühlkette ein hoher Energieaufwand erforderlich ist. Weiterhin gelangt das Probengefäß über einen Großteil seiner Oberfläche in direkten Kontakt mit der Atmosphäre des Arbeitsraums. Ferner ist es nicht zu vermeiden, dass ein geringer Anteil Umgebungsluft in den Arbeitsraum gelangt. Durch beide Umstände kann es zu nachteiliger Eisbildung im Arbeits- und Zwischenlager kommen.

WO 2005/010499 A2 offenbart ein Kryolagersystem zur Aufnahme, Speicherung und Ausgabe von Proben umfassend einen thermisch isolierten Kryoraum mit darin befindlichen Probenträgern zur Aufnahme der Proben und einem verschließbaren ersten Zugang in einem oberen Deckenbereich des Kryoraums, über die der Kryoraum mit einem über dem Kryoraum angeordneten thermisch isolierten Zwischenlager in Verbindung steht; mit Mitteln zum Transport mindestens eines Probenträgers zwischen dem Kryoraum und dem Zwischenlager; mit einem seitlich an das Zwischenlager anschließenden Arbeitsraum, in dem Mittel zum Transport in mindestens einer horizontalen Richtung einzeln aus dem Probenträger entnehmbarer und wieder in diesen einsteckbarer Elemente eines im Zwischenlager befindlichen Probenträgers in den Arbeitsraum angeordnet sind, wobei - in dem Kryoraum eine Probenträgeraufnahme zur Aufnahme mehrerer Probenträger vorhanden ist; - die Probenträgeraufnahme beweglich gelagert und mit einem gesteuerten Antrieb in Verbindung steht, so dass die einzelnen Probenträger dem ersten Zugang des Kryoraums zustellbar sind; - das Zwischenlager und der Arbeitsraum miteinander über einen zweiten Zugang in Verbindung stehen; - der Boden des Arbeitsraums mindestens über einen Bereich von unten und von außerhalb des Kryoraums zugänglich ist und in dem Boden des Arbeitsraums ein dritter Zugang vorhanden ist und Proben zwischen einem Probengefäß und dem Arbeitsraum transportiert werden können.

Der Erfindung liegt die Aufgabe zugrunde, ein Kryolagersystem vorzuschlagen, bei der die Kühlkette bei gegenüber dem Stand der Technik reduziertem Energieaufwand ununterbrochen gewahrt bleibt und die Gefahr einer unerwünschten Eisbildung verringert wird.

Die Aufgabe wird in einem Kryolagersystem zur Aufnahme, Speicherung und Ausgabe von Proben umfassend einen thermisch isolierten Kryoraum mit darin befindlichen Probenträgern zur Aufnahme der Proben und einem verschließbaren ersten Zugang in einem oberen Deckenbereich des Kryoraums, über den der Kryoraum mit einem über dem Kryoraum angeordneten thermisch isolierten Zwischenlager in Verbindung steht; mit Mitteln zum Transport mindestens eines Probenträgers zwischen dem Kryoraum und dem Zwischenlager; mit einem seitlich an das Zwischenlager anschließenden Arbeitsraum, in dem Mittel zum Transport in mindestens einer horizontalen Richtung einzeln aus dem Probenträger entnehmbarer und wieder in diesen einsteckbarer Elemente eines im Zwischenlager befindlichen Probenträgers in den Arbeitsraum angeordnet sind und in dem eine Schleuse zum Transport von Proben zwischen einem Probentransportgefäß und dem Arbeitsraum vorhanden ist, dadurch gelöst,
dass in dem Kryoraum eine Probenträgeraufnahme zur Aufnahme mehrerer Probenträger vorhanden ist;
dass die Probenträgeraufnahme beweglich gelagert und mit einem gesteuerten Antrieb in Verbindung steht, so dass die einzelnen Probenträger dem ersten Zugang des Kryoraums zustellbar sind;
dass das Zwischenlager und der Arbeitsraum gegeneinander thermisch isoliert sind; dass das Zwischenlager und der Arbeitsraum miteinander über einen verschließbaren zweiten Zugang in Verbindung stehen;
dass der Boden des Arbeitsraums mindestens über einen Bereich von unten und von außerhalb des Kryoraums zugänglich ist und in dem Boden des Arbeitsraums ein verschließbarer dritter Zugang mit einem Dichtungselement und einer Arretiervorrichtung zur lösbaren Arretierung eines Probengefäßes derart vorhanden sind, dass das Probengefäß gasdicht an dem dritten Zugang des Arbeitsraums arretierbar ist und Proben zwischen einem arretierten Probengefäß und dem Arbeitsraum transportiert werden können.

Ein Kryoraum ist vorteilhaft als ein zylindrischer Behälter mit isolierten Wänden ausgebildet, dessen Boden nach außen gewölbt ist. Er ist mittels geeigneter Kühlvorrichtungen auf tiefkalte Temperaturen gesteuert kühlbar. Als "tiefkalt" werden nachfolgend Temperaturen bezeichnet, die wesentlich (z. B. 30 K) unter der Temperatur der Umgebung liegen. Je nach Situation und verwendeter Definition können dies Temperaturen um den Gefrierpunkt bis hin zum absoluten Nullpunkt sein. Tiefkalte Temperaturen können in bekannter Weise durch die Wirkung eines Kühlmediums, wie z. B. flüssiger und verdampfender Stickstoff, erzeugt sein.

Unter biologischen Proben werden nachfolgend Zellproben wie beispielsweise Zellsuspensionen, Suspensionen oder Lyophilisate organischer Stoffe oder Stoffverbindungen, Lysate, Gewebe oder Gewebteile, Organe, Organellen und molekulare Träger von Erbinformationen wie DNS oder RNS und deren Kombination verstanden.

Probenträger können jegliche Vorrichtungen sein, in denen Proben gehalten werden können. Dabei sind die Proben üblicherweise in Gefäßen mit genormten Abmaßen enthalten, wie diese in einem nach dem Stand der Technik ausgerüsteten Labor verwendet werden. Die Probenträger können beispielsweise sogenannte Racks sein. In einer vorteilhaften Ausführung der erfindungsgemäßen Vorrichtung sind die entnehmbaren und wieder in den Probenträger einsteckbaren Elemente der Probenträger einzelne Schubladen, welche jeweils eine Grundfläche (Querschnittsfläche orthogonal zur Längsachse des Zylinders) aufweisen, die im Wesentlichen einem Zylindersektor entsprechen.

Bevorzugt entsprechen die Grundflächen der Schubladen der Grundfläche eines Zylinders, der in Zylindersektoren von einem Viertel bzw. von einem Sechstel geteilt ist. In weiteren Ausführungen, die nicht Teil der Erfindung sind, können die Grundflächen auch z. B. rechteckig, rund oder trapezförmig sein.

Eine solche Gestaltung der Schubladen ist dann besonders vorteilhaft, wenn auch der Probenträger eine Grundfläche eines Zylindersektors aufweist. Besitzen die Probenträger die Grundfläche eines Zylindersektors, können diese platzsparend nahe um eine zentrale Achse in dem Kryoraum angeordnet sein. Zudem ist es möglich, die Dimensionierung des ersten Zugangs, über die der Kryoraum mit dem Zwischenlager in Verbindung steht, gering zu halten. Vorzugsweise ist die Form und Größe des ersten Zugangs korrespondierend zu der Form und Größe der Grundfläche des Probenträgers ausgeführt, so dass beim Transport des Probenträgers zwischen dem Kryoraum und dem Zwischenlager nur ein geringer Volumenstrom eines Kühlmediums auftritt.

Um die Probenträgeraufnahme mit den Probenträgern dem ersten Zugang in einer einfachen Weise zustellen zu können, ist die Probenträgeraufnahme vorteilhaft um eine Drehachse drehbar ausgeführt. Dabei kann die Probenträgeraufnahme um eine koaxial der Drehachse verlaufende Welle angeordnet und mit dieser verbunden sein, wobei die Welle an ihrem im Kryoraum befindlichen Ende in einem Lager um die Drehachse drehbar gelagert ist. Das Lager kann dabei beispielsweise ein Axiallager, ein Radiallager oder ein Radiaxlager sein.

Die Welle ist mit einem Antrieb verbunden und mittels des Antriebs gesteuert drehbar. Der Antrieb ist vorzugsweise in einem Bereich, innerhalb oder außerhalb des Kryoraums bzw. des Zwischenlagers angeordnet, in dem Temperaturen wärmer als -40°C vorliegen. Alternativ kann der Antrieb beheizbar ausgeführt sein.

Um eine gleichmäßige horizontale Ausrichtung der Probenträger während deren Zustellung zu dem ersten Zugang zu gewährleisten, ist die Probenträgeraufnahme vorteilhafterweise an mehreren Punkten gegen eine Wand oder den Boden des Kryoraums abgestützt.

In einer vorteilhaften Ausführung des Kryolagersystems, die nicht Teil der Erfindung ist, ist die Probenträgeraufnahme mit ihrer Unterseite auf Bockrollen abgestützt, welche aus Richtung des Bodens des Kryoraums in Richtung der Unterseite der Probenträgeraufnahme ragen.

Die Bockrollen können am Boden des Kryoraums befestigt sein und in Richtung der Unterseite der Probenträgeraufnahme nach oben ragen. Es ist auch möglich, dass über dem Boden des Kryoraums eine oder mehrere Befestigungselemente für die Bockrollen, z. B. in Form von kreis- oder ringförmigen Schienen, vom Boden aufragende Stempel oder Vorsprünge an der Wand des Kryoraums, angeordnet sind. Die Bockrollen können starr in einem festen Winkel angeordnet sein. Es ist auch möglich, dass eine die jeweiligen Bockrollen haltende Lagerung jeweils um eine vertikale Achse drehbar gestaltet ist.

Es ist eine bevorzugte Ausführung, wenn jeder Probenträger Elemente zur Erzielung einer lösbaren Verbindung, beispielsweise Kupplungselemente zur Bildung einer Kupplung, aufweist, mittels dem je ein Probenträger an ein Hebeelement angekuppelt und zwischen Kryoraum und dem Zwischenlager transportiert werden kann.

In einer Ausführung, die nicht Teil der Erfindung ist, ist ein Hebelement zugleich als Verschluss des ersten Zugangs (erster Verschluss) ausgebildet. Der erste Verschluss weist an seiner in den Kryoraum weisenden Seite erste Kupplungselemente auf. Jeder Probenträger weist zweite Kupplungselemente auf, wobei durch die ersten Kupplungselemente und die zweiten Kupplungselemente eine Kupplung zur lösbaren Verbindung des ersten Verschlusses und einem, dem ersten Zugang zugestellten, Probenträger gebildet ist, mittels der je ein an dem ersten Verschluss angekuppelter Probenträger zwischen dem Kryoraum und dem Zwischenlager transportierbar ist.

Das Hebeelement kann über eine Kette mit einem Kettenantrieb verbunden sein. Die Verwendung einer Kette erlaubt eine verbesserte Wiederholgenauigkeit von vertikalen Bewegungen eines an das Hebelement angekuppelten Probenträgers. Das Zwischenlager besitzt in einer vorteilhaften, weil energiesparenden Ausführung, eine Dimensionierung, bei der die gleichzeitige Aufnahme immer nur eines Probenträgers ermöglicht ist.

Der zweite Zugang zwischen Zwischenlager und Arbeitsraum weist vorzugsweise eine vertikale Ausdehnung auf, über deren Bereich Schubladen horizontal zwischen dem Zwischenlager und dem Arbeitsraum transportierbar sind. Die vertikale Ausdehnung des zweiten Zugangs endet beabstandet zu den jeweiligen Deckenbereichen des Zwischenlagers und des Arbeitsraums.

Durch eine solche Gestaltung wird in äußerst vorteilhafter Weise sowohl in dem Zwischenlager als auch in dem Arbeitsraum oberhalb einer Oberkante des zweiten Zugangs und dem jeweiligen Deckenbereich des Zwischenlagers, respektive des Arbeitsraums, je ein Volumen geschaffen, in dem eine vertikale Temperaturschichtung einstellbar ist, die weitgehend unabhängig von der Temperaturschichtung in dem jeweils anderen Volumen ist. Dabei wird der Umstand ausgenutzt, dass sich bei fehlenden oder nur gering ausgeprägten Volumenströmungen wärmere Anteile eines Mediums mit einer geringeren spezifischen Dichte, hier eines Kühlmediums, wie z. B. Stickstoff oder ein Stickstoff-Luft-Gemisch, über kälteren Anteilen, welche eine höhere spezifische Dichte aufweisen, lagert. Da die üblicherweise als Kühlmedien in Kryolagersystemen verwendeten Gase oder Gasgemische schlechte Wärmeleiter sind, kann in jedem der Volumina ab der Oberkante des zweiten Zugangs ein vertikaler Temperaturverlauf eingestellt werden, der weitgehend unabhängig von einem vertikalen Temperaturverlauf des jeweils anderen Volumens ist.

Es ist ermöglicht, dass über die vertikale Ausdehnung des zweiten Zugangs in dem Zwischenlager und dem Arbeitsraum ein gleicher vertikaler Temperaturverlauf eingestellt ist. In dem oberhalb des zweiten Zugangs befindlichen Volumens des Zwischenlagers können geringere Temperaturen, z. B. ≤ -100°C, eingestellt sein als oberhalb des zweiten Zugangs in dem Volumen des Arbeitsraums, z. B. ≤ - 10°C. Dies trifft auch dann zu, wenn die Höhe der Volumina in vertikaler Richtung oberhalb des zweiten Zugangs nicht gleich sind. Es ist möglich, in beiden Volumina unterschiedliche vertikale Temperaturverläufe, z. B. als lineare Temperaturgradienten (Temperaturänderung je Schritt in vertikaler Richtung), zu schaffen.

Die vertikalen Temperaturverläufe, insbesondere der vertikale Temperaturverlauf in dem Arbeitsraum, ist durch die Dauer und den Grad der Öffnung des zweiten Verschlusses regelbar.

Die beschriebene Gestaltung erlaubt es beispielsweise, in dem Volumen des Arbeitsraums oberhalb des zweiten Zugangs Vorrichtungen zu installieren, die für ihre zuverlässige Funktionsfähigkeit bei Temperaturen wärmer als -40°C zu betreiben sind.

Es ist ferner ein Vorteil des erfindungsgemäßen Kryolagersystems, dass Proben, die sich in einem Probenträger im Zwischenlager befinden, auch dann bei beispielsweise mindestens -100°C gelagert sind, wenn der zweiten Zugang geöffnet ist.

Weiterhin sind Proben, die auf Höhe des zweiten Zugangs in dem Arbeitsraum vorhanden sind, ebenfalls bei beispielsweise mindestens -100°C gelagert, so dass bei keiner in einem Probenträger enthaltenen Probe eine Unterbrechung der Kühlkette auftritt. Zudem sind die Proben keinen nachteiligen wesentlichen Temperaturschwankungen ausgesetzt.

Um die genannten Vorteile der Gestaltung des zweiten Zugangs effizient nutzen zu können, ist es von Vorteil, wenn die Schubladen eines im Zwischenlager befindlichen Probenträgers, durch Positionieren des Probenträgers, einer Übergabeposition zustellbar sind und die Mittel zum Transport der Schubladen zwischen einem im Zwischenlager befindlichen Probenträger und dem Arbeitsraum der Übergabeposition zustellbar sind, wobei die Übergabeposition so definiert ist, dass ein Transport von Schubladen durch den zweiten Zugang möglich ist.

Eine Positionierung des Probenträgers erfolgt in einer einfachen Ausführung, die nicht Teil der Erfindung ist, durch die Mittel wie Antrieb, Kette und Hebeelement, mit denen der Probenträger gesteuert vertikal in dem Zwischenlager bewegbar ist.

Um einen Einfluss von temperaturabhängigen Ausdehnungen und Schrumpfungen auf die Genauigkeit der Zustellung der Mittel zum Transport der Schubladen zur Übergabeposition sowie der Transportbewegung, insbesondere auf die Genauigkeit der Einhaltung von End- und ggf. auch von Wendepunkten weitgehend zu reduzieren, sind die Mittel zum Transport der Schubladen zwischen dem im Zwischenlager befindlichen Probenträger und dem Arbeitsraum vorteilhaft eine Transportvorrichtung, die mittels eines Zugmittels angetrieben ist. Zugmittel sind Mittel zur Übertragung von Zugkräften wie beispielsweise Riemen, Seile oder Ketten. In einer vorteilhaften Ausführung der Erfindung ist als Zugmittel eine Kette vorhanden. In einer bevorzugten Ausführung des Kryolagersystems ist die Transportvorrichtung so gestaltet, dass diese eine in den Arbeitsraum transportierte Schublade einer Zugriffsposition mit definierten Lagekoordinaten im Arbeitsraum zustellbar ist. Durch eine Zustellung der Schublade in eine Zugriffsposition ist eine Manipulation von Proben durch automatische Manipulationsvorrichtungen, wie z. B. ein Robotersystem, wesentlich erleichtert, da keine fortwährenden Ist-Soll-Vergleiche der Lagekoordinaten der Schublade und der Manipulationsvorrichtung erforderlich sind.

Eine Vielzahl von Proben in einem Kryolagersystem erfordert für einen effektiven Betrieb der Einrichtung eine Möglichkeit zur eindeutigen und individuellen Lokalisierung der Proben. Es ist daher eine vorteilhafte Ausführung, wenn an jeder Schublade Speichermittel zur Speicherung und Bereitstellung von Daten, beispielweise ein aktiver Sender oder ein passiver Transponder, vorhanden sind. Zudem kann jeder Probenträger mit einem Speichermittel versehen sein.

Zur Erfassung der Daten können in dem Arbeitsraum Kontaktierungsmittel zur Kontaktierung der Speichermittel einer in den Arbeitsraum transportierten Schublade vorhanden sein, wobei durch die Kontaktierung Daten zwischen dem Kontaktierungsmittel und dem Speichermittel austauschbar sind. Kontaktierungsmittel können z. B. berührungslos arbeitende Sensoren wie optische Barcodescanner, Kameras oder Transponderlesegeräte zum Anregen und Auslesen passiver Transponder sein. Eine Verwendung von taktil arbeitenden Kontaktierungsmitteln, z. B. von Steckkontakten, ist ebenfalls allein oder in Kombination mit berührungslos arbeitenden Sensoren möglich, ebenso eine Anordnung von Kontaktierungsmitteln außerhalb des Arbeitsraums.

Vorteilhaft sind die Kontaktierungsmittel sowie die verschiedenen genannten Antriebe und Vorrichtungen mit Mitteln zur Speicherung und Verwaltung von Informationen, z. B. mit Rechner- und Steuereinheiten, verbunden.

Es ist weiterhin günstig, wenn in dem Arbeitsraum eine gesteuert arbeitende Manipulationsvorrichtung zur Manipulation von Proben durch den dritten Zugang und im Arbeitsraum vorhanden ist. Eine solche Manipulationsvorrichtung kann eine manuell zu bedienende Vorrichtung, wie beheizbare Handschuhe an den Eingriffen oder ein halb- oder vollautomatisch arbeitendes System wie ein Roboter zur Entnahme, Manipulation und Einbringung einer oder mehrerer Proben sein.

Durch eine lösbare Arretierung des Probengefäßes an der Arretiervorrichtung an dem Boden des Arbeitsraums wird äußerst vorteilhaft vermieden, dass die vergleichsweise warme Außenwand des Probengefäßes in den Arbeitsraum gelangt und sich aufgrund der dort herrschenden tiefkalten Temperaturen Kondensat oder Eis bildet. Zudem wird ein Eintreten warmer und feuchtigkeitsbeladener Umgebungsluft in den Arbeitsraum verhindert.

Das Probengefäß kann manuell oder aber automatisch der Arretiervorrichtung zugestellt werden. Das Dichtungselement kann in einer vorteilhaften Ausführung beheizbar sein, so dass eine gasdichte Arretierung des Probengefäßes an der Arretiervorrichtung sowie eine leichtgängige Funktionsweise der Arretiervorrichtung gewährleistet ist.

Es ist ferner möglich, eine Anzahl erfindungsgemäßer Kryolagersysteme anzuordnen und dieser ganz oder teilweise gesteuert, z. B. über eine zentrale oder dezentrale Datenbank zu steuern, Proben zuzuführen und zu entnehmen sowie um Proben innerhalb der Schubladen, innerhalb eines Probenträgers, zwischen verschiedenen Probenträgern oder zwischen verschiedenen Kryolagersystemen zu verlagern und dabei gleichzeitig den aktuellen Ort der Lagerung einer individuellen Probe zu dokumentieren.

Die Erfindung wird nachfolgend anhand von Ausführungsbeispielen und Abbildungen näher erläutert. Die Abbildungen zeigen:
- Fig. 1: eine erste Ausführung des erfindungsgemäßen Kryolagersystems in einem ersten Betriebszustand im medianen Längsschnitt;
- Fig. 2: die erste Ausführung des erfindungsgemäßen Kryolagersystems in einem zweiten Betriebszustand im medianen Längsschnitt;
- Fig. 3: eine zweite Ausführung des erfindungsgemäßen Kryolagersystems in einer horizontalen Schnittdarstellung und
- Fig. 4: eine Darstellung eines Dichtungselements an einem dritten Zugang des erfindungsgemäßen Kryolagersystems.

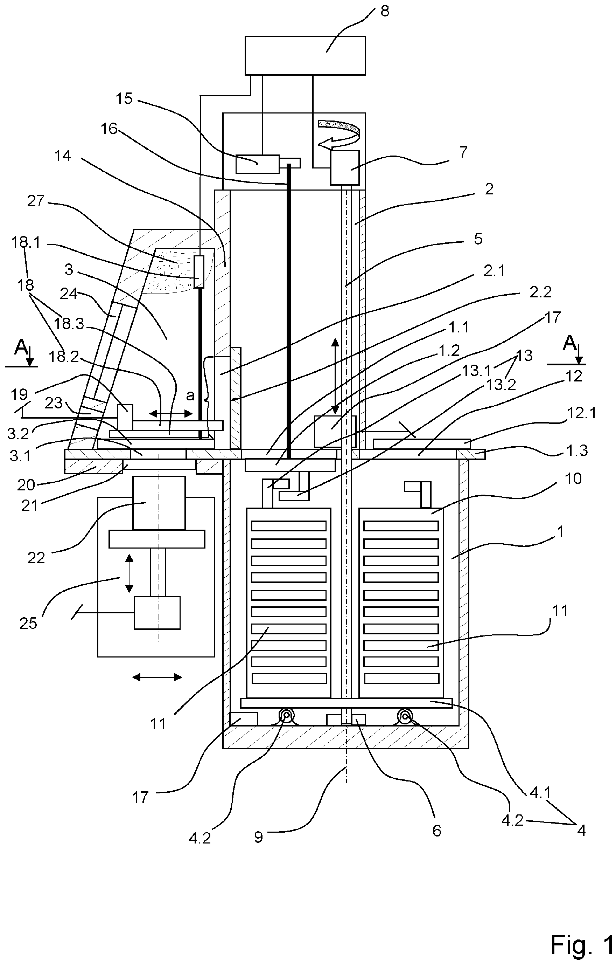

Als wesentliche Elemente einer Ausführung des erfindungsgemäßen Kryolagersystems sind in der Fig. 1 ein Kryoraum 1 mit einer Probenträgeraufnahme 4 und einem ersten Zugang 1.1 in einer den Kryoraum 1 nach oben abschließenden Kryoraumdecke 1.3, ein über dem Kryoraum 1 angeordnetes Zwischenlager 2 und ein horizontal neben dem Zwischenlager 2 befindlicher Arbeitsraum 3 mit einem in seinem Boden befindlichen dritten Zugang 3.1 gezeigt, wobei die Räume jeweils von wärmeisolierten Wänden umschlossen sind.

In den Kryoraum 1 ragt von senkrecht oben eine Welle 5 hinein, die am Boden des Kryoraums 1 in einem Lager 6 um eine Drehachse 9, die mit der Symmetrielängsachse der Welle 5 zusammenfällt, drehbar gelagert ist. Die Probenträgeraufnahme 4 ist als eine Platte 4.1 ausgebildet, die horizontal ausgerichtet und von dem Boden des Kryoraums 1 beabstandet so um die Welle 5 angeordnet ist, dass die Platte 4.1 durch die Welle 5 um die Drehachse 9 drehbar ist. Die Platte 4.1 ist mit der Unterseite der Platte 4.1 auf an dem Boden des Kryoraums 1 angebrachten und in Richtung der Unterseite der Platte 4.1 ragenden Bockrollen 4.2 abgestützt. Die Bockrollen 4.2 sind so angebracht und ausgerichtet, dass die Platte 4.1 während einer Bewegung um die Drehachse 9 geführt und gestützt ist. Auf der Platte 4.1 sind lösbar Probenträger 10 aufgesetzt, deren Grundfläche einem Zylindersektor entspricht. Die Abmaße der zylindersektorförmigen Grundfläche sind so gewählt, dass eine Anzahl von Probenträgern 10 einen Kreis um die Drehachse 9 bildend angeordnet sein können. Zur Vereinfachung der Darstellung sind nur zwei Probenträger 10 gezeigt. In der Nähe des Bodens des Kryoraums 1 ist eine Kühleinrichtung 17 zur Einstellung tiefkalter Temperaturen in dem Kryoraum 1, z. B. durch Zufuhr eines tiefkalten Kühlmediums wie flüssigen Stickstoffs, vorhanden.

Die Probenträger 10 sind als Rahmen, sogenannte Racks, gestaltet, in denen übereinander horizontal ausgerichtete Schubladen 11 zur Aufnahme von Proben vorhanden sind. Die einzelnen Schubladen 11 sind horizontal aus dem Probenträger 10 entnehmbar. An dem oberen Bereich des Probenträgers 10 ist ein erstes Kupplungselement 13.1 als ein umgekehrt L-förmiges Profil befestigt.

Der Kryoraum 1 steht über den ersten Zugang 1.1 mit dem Zwischenlager 2 in Verbindung. Der erste Zugang 1.1 weist eine zylindersektorförmige Form und eine solche Größe auf, dass ein Probenträger 10 durch den ersten Zugang 1.1 hindurch transportierbar ist. Der erste Zugang 1.1 ist durch einen ersten Verschluss 1.2 verschließbar. An der in den Kryoraum 1 weisenden Seite des ersten Verschlusses 1.2 ist ein zweites Kupplungselement 13.2 vorhanden, das ebenfalls als ein umgekehrt L-förmiges Profil ausgebildet ist. Durch das erste Kupplungselement 13.1 eines Probenträgers 10 und dem zweiten Kupplungselement 13.2 ist eine Kupplung 13 gebildet. Bei einer Zustellung eines Probenträgers 10 unter den ersten Zugang 1.1 ragen die freien Schenkel des ersten und des zweiten Kupplungselements 13.1, 13.2 ineinander, ohne sich zu berühren.

Eine Zustellung eines Probenträgers 10 unter den ersten Zugang 1.1 erfolgt gesteuert motorisch. In weiteren Ausführungen der Erfindung kann die Zustellung auch manuell erfolgen.

Das über dem Kryoraum 1 befindliche Zwischenlager 2 weist eine Grundfläche auf, die kleiner als die Grundfläche des Kryoraums 1 ist, so dass ein Teil der Kryoraumdecke 1.3 nicht von dem Zwischenlager 2 überdeckt ist. In dem nicht überdeckten Bereich der Kryoraumdecke 1.3 ist ein Wartungszugang 12 vorhanden, der mittels eines Verschlusses 12.1 des Wartungszugangs 12 verschließbar ist. Durch den Wartungszugang 12 sind die Probenträger 10, z. B. im Fall einer Havarie des erfindungsgemäßen Kryolagersystems, manuell oder maschinell entnehmbar.

An der in das Zwischenlager 2 weisenden Seite des ersten Verschlusses 1.2 ist eine Kette 16 befestigt, die mit einem Kettenantrieb 15, der oberhalb und außerhalb des Zwischenlagers 2 angeordnet ist, verbunden ist. Bei einer Anhebung des ersten Verschlusses 1.2 mittels des Kettenantriebs 15 und der Kette 16 gelangen das erste Kupplungselement 13.1 und das zweite Kupplungselement 13.2 in formschlüssigen Kontakt. Der an dem ersten Verschluss 1.2 angekuppelte Probenträger 10 ist mitsamt dem ersten Verschluss 1.2 durch den ersten Zugang 1.1 in das Zwischenlager 2 transportierbar.

In dem gezeigten ersten Betriebszustand ist der erste Zugang 1.1 durch den ersten Verschluss 1.2 verschlossen und es befinden sich beide Probenträger 10 auf der Probenträgeraufnahme 4.

Die Abmaße des Zwischenlagers 2 sind so bemessen, dass nur jeweils ein Probenträger 10 in ihm Platz findet. In dem Zwischenlager 2 ist in der Nähe des ersten Zugangs 1.1 eine regelbare Kühleinrichtung 17 zur Kühlung und zur Regelung der Temperatur des Zwischenlagers 2 vorhanden.

Durch das Zwischenlager 2 reicht die Welle 5 von einem oben in dem Zwischenlager 2 befindlichen Antrieb 7 in den Kryoraum 1. Der Antrieb 7 kann in weiteren Ausführungen auch außerhalb des Zwischenlagers 2 angeordnet sein.

Das Zwischenlager 2 und der Arbeitsraum 3 sind durch eine vertikale Trennwand 14, die wärmeisoliert ausgeführt ist, gegeneinander abgegrenzt. Die Böden des Zwischenlagers 2 und des Arbeitsraums 3 befinden sich horizontal auf einer Höhe. In der Trennwand 14 ist ein zweiter Zugang 2.1 vorhanden, der eine vertikale Ausdehnung a aufweist und in der unteren Hälfte der Trennwand 14 angeordnet ist. Der zweite Zugang 2.1 ist durch einen zweiten Verschluss 2.2 in Form eines ansteuerbaren und maschinell bewegbaren Schiebers verschließbar.

In dem Arbeitsraum 3, dessen vertikale Ausdehnung sich über eine Oberkante des zweiten Zugangs 2.1 hinaus erstreckt, ist eine Transportvorrichtung 18 zum Transport von Schubläden 11 bei geöffnetem zweiten Verschluss 2.2 zwischen dem Zwischenlager 2 und dem Arbeitsraum 3 durch den zweiten Zugang 2.1 hindurch vorhanden. Die Transportvorrichtung 18 umfasst einen Antrieb 18.1, durch den über eine weitere Kette 16 eine Transportplattform 18.2 in einer Transportebene 18.3 angetrieben wird. Durch die Transportvorrichtung 18 ist der zweite Verschluss 2.2 betätigt.

Ferner ist ein Kontaktierungsmittel 19 so angeordnet, dass an den Proben und/oder den in den Arbeitsraum 3 transportierten Schubläden 11 vorhandenen Speichermittel (nicht gezeigt) kontaktiert und die in den kontaktierten Speichermitteln enthaltenen Daten erfassbar und veränderbar sind.

Durch die Transportvorrichtung 18 ist eine in den Arbeitsraum 3 transportierte Schublade 11 in eine Zugriffsposition mit definierten Lagekoordinaten gebracht.

In einer weiteren Ausführung des erfindungsgemäßen Kryolagersystems kann die Transportvorrichtung 18 so ausgebildet sein, dass Schubläden 11 innerhalb des Arbeitsraums 3 verfahrbar sind und abgelegt werden können. Eine derart abgelegte Schublade 11 kann in eine (zweite) Zugriffsposition gebracht sein.

Eine dem Zwischenlager 2 angewandte Wand des Arbeitsraums 3 ist schräg nach oben in Richtung auf das Zwischenlager 2 zu geneigt und weist ein Sichtfenster 24 sowie zwei mit beheizten Handschuhen (nicht gezeigt) versehene Eingriffe 23 zum Manipulation von Proben durch eine Bedienperson in dem Arbeitsraum 3 auf.

In dem Arbeitsraum 3 ist ein vertikaler Temperaturverlauf so eingestellt, dass über die vertikale Ausdehnung des zweiten Zugangs 2.1 Temperaturen vorliegen, die nicht wärmer als die in dem Zwischenlager 2 eingestellten Temperaturen (Arbeitstemperatur, z. B. -100°C) sind. Über die vertikale Ausdehnung des eine gewisse Dauer geöffneten zweiten Zugangs 2.1 sind die Temperaturen der vertikalen Temperaturverläufe in dem Arbeitsraum 3 und dem Zwischenlager 2 gleich. Über die Dauer und den Grad der Öffnung des zweiten Zugangs 2.1 ist der vertikale Temperaturverlauf in dem Arbeitsraum 3 regelbar.

Ab der Oberkante des zweiten Zugangs 2.1 steigen die Temperaturen in dem Arbeitsraum 3 in vertikaler Richtung schneller an als in dem Zwischenlager 2. An einem Deckenbereich des Arbeitsraums 3 liegen Temperaturen wärmer als -40°C vor (Warmbereich 27), während auf der gleichen Höhe die Temperaturen in dem Zwischenlager 2 beispielsweise -80°C betragen. Die vertikalen Temperaturverläufe in dem Zwischenlager 2 und dem Arbeitsraum 3 sind ab der Oberkante des zweiten Zugangs 2.1 voneinander verschieden und im Wesentlichen voneinander unabhängig einstellbar. Die Erzeugung des Warmbereichs 27 in dem Arbeitsraum 3 erlaubt die Anordnung aller mechanisch arbeitenden Komponenten der Transportvorrichtung 18 innerhalb des Arbeitsraums 3.

In weiteren Ausführungen des erfindungsgemäßen Kryolagersystems können Heizvorrichtungen vorhanden sein, durch die eine Einstellung der vertikalen Temperaturverläufe möglich ist.

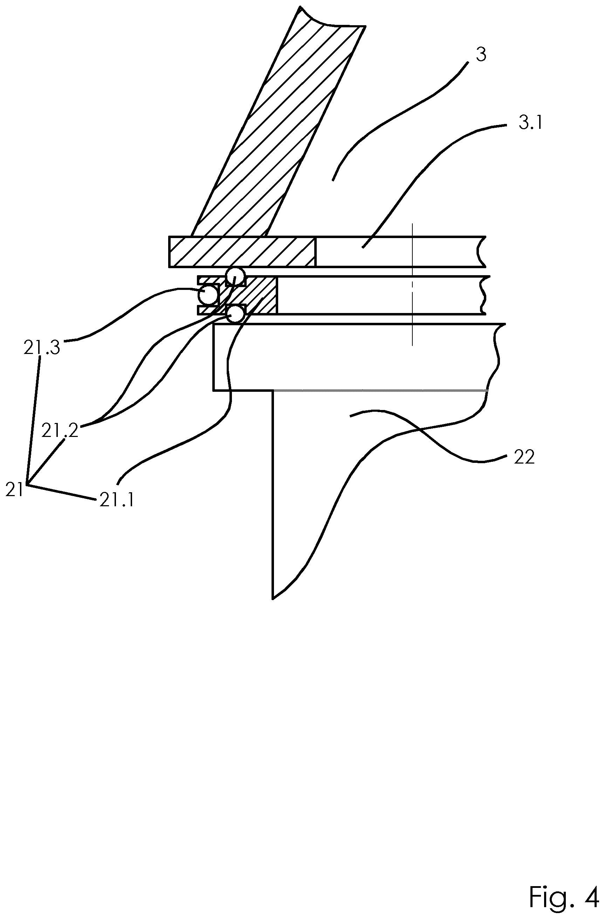

In dem Boden des Arbeitsraums 3 befindet sich neben der Transportvorrichtung 18 ein dritter Zugang 3.1, durch den ein Zugang von senkrecht unten in den Arbeitsraum 3 ermöglicht ist. Der dritte Zugang 3.1 ist durch einen dritten Verschluss 3.2 verschließbar. Um den dritten Zugang 3.1 ist eine Arretiervorrichtung 20 angebracht, an der ein als Dewargefäß ausgebildetes Probengefäß 22 arretierbar ist. Um eine Dichtheit zwischen einem arretierten Probengefäß 22 und dem Arbeitsraum 3 zu gewährleisten, ist ein Dichtungselement 21, dass durch ein Heizelement 21.3 (siehe Fig. 4) auf Temperaturen wärmer als -40°C heizbar ist, vorhanden.

Das Probengefäß 22 ist dem dritten Zugang 3.1 mittels einer Zustellvorrichtung 25 zustellbar. Diese kann einzeln betrieben oder beispielsweise in ein automatisch arbeitendes Netzwerk mehrerer Kryolagersysteme eingebunden sein.

Das Kryolagersystem ist durch eine Steuerung 8 gesteuert. Die Steuerung 8 ist mit dem zweiten Verschluss 2.2, dem Antrieb 7, dem Kettenantrieb 15, der Kühleinrichtung 17, dem Antrieb 18.1 der Transportvorrichtung 18, dem Kontaktierungsmittel 19 und der Zustellvorrichtung 25 signalleitend verbunden.

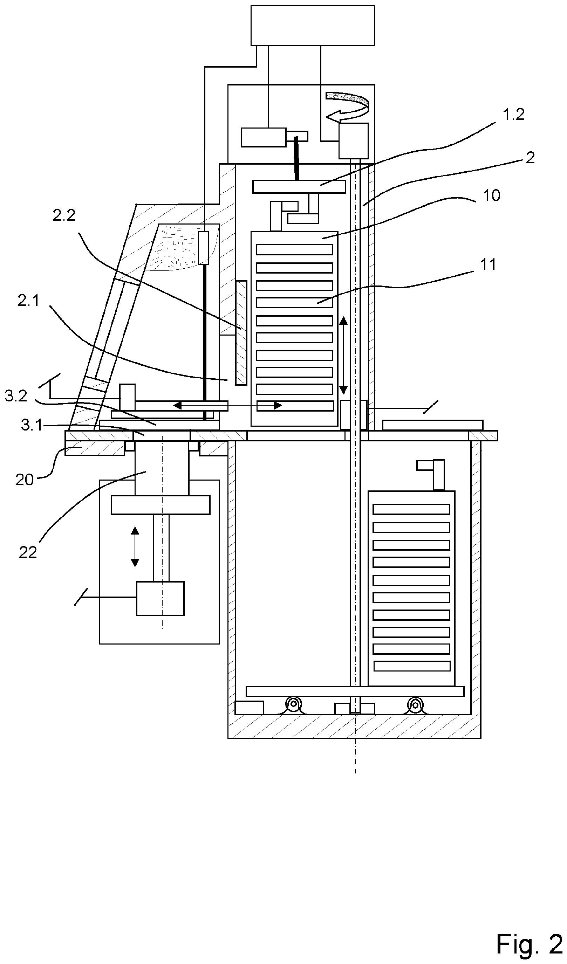

In dem in Fig. 2 gezeigten zweiten Betriebszustand ist der Probenträger 10 angekuppelt und in das Zwischenlager 2 transportiert. Der zweite Verschluss 2.2 ist angehoben und eine Schublade 11 aus dem Probenträger 10 durch den zweiten Zugang 2.1 hindurch in den Arbeitsraum 3 transportiert.

Das Probengefäß 22 ist an der Arretiervorrichtung 20 um den dritten Zugang 3.1 arretiert und der dritte Verschluss 3.2 ist geöffnet.

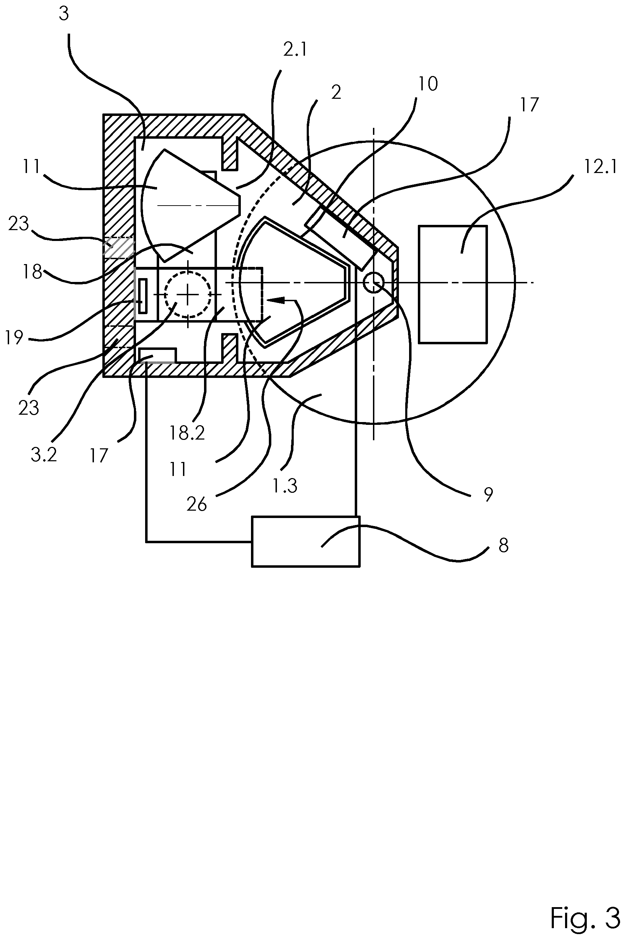

Eine zweite Ausführung des erfindungsgemäßen Kryolagersystems ist in der Fig. 3 als horizontaler Schnitt durch das Zwischenlager 2 und den Arbeitsraum 3 oberhalb der Transportvorrichtung 18 und durch den geöffneten zweiten Zugang 2.1 gezeigt. Die Grundfläche des Zwischenlagers 2 weist die Form eines ungleichschenkligen Trapezes auf, ist so dimensioniert, dass nur ein Probenträger 10 in dem Zwischenlager 2 Platz findet und überdeckt nur einen Teilbereich der die Decke des Kryoraums 1 bildenden Kryoraumdecke 1.3. Die Temperatur sowie eine vertikale Temperaturschichtung in dem Zwischenlager 2 ist mittels der Kühleinrichtung 17 regelbar.

In das Zwischenlager 2 ist ein Probenträger 10 aus dem unter dem Zwischenlager 2 angeordneten Kryoraum 1 transportiert worden. Stark vereinfacht gezeigt ist eine in dem Probenträger 10 befindliche Schublade 11, die sich auf der Höhe des zweiten Zugangs 2.1 befindet und einer Übergabeposition 26 in der Transportebene 18.3 (nicht gezeigt) zugestellt ist. Die Übergabeposition 26 (durch einen Pfeil symbolisiert) ist durch eine relative Winkellage des in den Zwischenlager 2 transportierten Probenträgers 10 um die Drehachse 9 und einer vertikalen Position bestimmt, die es ermöglichen, dass eine Entnahme oder ein Hineinbringen einer Schublade 11 durch die Transportplattform 18.2 aus bzw. in den Probenträger 10 möglich ist. Eine weitere Schublade 11 ist durch die Transportvorrichtung 18 aus dem Probenträger 10 entnommen, durch den zweiten Zugang 2.1 in den Arbeitsraum 3 transportiert und in dem Arbeitsraum 3 seitlich des zweiten Zugangs 2.1 abgelegt. Das Kontaktierungsmittel 19 ist gegenüber dem zweiten Zugang 2.1 so angeordnet, dass eine mittels der Transportplattform 18.2 zwischen dem Arbeitsraum 3 und dem Zwischenlager 2 transportierten Schublade 11 und/oder die darin befindlichen Proben kontaktierbar sind.

In dem gezeigten Betriebszustand ist es möglich, aus einem an der Arretiervorrichtung 20 arretierten Probengefäß 22 durch den dritten Zugang 3.1 Proben zwischen dem Arbeitsraum 3 und dem Probengefäß 22 zu transportieren.

Beispielsweise könnten Proben aus dem Probengefäß 22 entnommen und in die im Arbeitsraum 3 abgelegte Schublade 11 einsortiert werden.

In dem Arbeitsraum 3 ist eine weitere Kühleinrichtung 17 angeordnet. Diese ist mit der Steuerung 8 verbunden. Die tatsächlichen Kühlleistungen der Kühleinrichtungen 17 in dem Zwischenlager 2 und dem Arbeitsraum 3 sind durch die Steuerung 8 aufeinander abstimmbar. Es ist ferner möglich, dass in mindestens einem der Räume Kryoraum 1, Zwischenlager 2 und Arbeitsraum 3 mindestens ein Temperatursensor vorhanden ist, der mit der Steuerung 8 in Verbindung steht.

Eine Ausführung eines Dichtungselements 21 ist gemäß der Fig. 4 als Schnitt gezeigt. Um den dritten Zugang 3.1 umlaufend ist auf der nach außen weisenden Seite des Boden des Arbeitsraums 3 eine Halterung 21.1 aus gut wärmeleitendem Material angeordnet. Zwei runde Dichtungsgummis 21.2 sind in Nuten der Halterung 21.1 so eingelegt, dass ein Dichtungsgummi 21.2 nach oben weist und in Kontakt zu dem Boden des Arbeitsraums 3 und der andere Dichtungsgummi 21.2 nach unten weist und in Kontakt zu einem oberen Rand des arretierten Probengefäßes 22 steht. In einer weiteren, radial gerichteten Nut ist ein Heizelement 21.3 eingelegt, welches hinsichtlich seiner Heizleistung durch die Steuerung 8 (hier nicht gezeigt) geregelt ist.

In einer weiteren Ausführung der Erfindung ist die Halterung 21.1 gegen einen tiefkalten Innenraum des Probengefäßes 22 kälteisoliert.

Über Zeiträume, über die das Probengefäß 22 nicht arretiert ist, ist stattdessen ein wärmeisolierter Ersatzkörper, ein sogenannter Dummy, arretiert. Der nach unten weisende Dichtungsgummi 21.2 tritt dann mit einem Rand des Dummys in Kontakt.

Der Betrieb eines erfindungsgemäßen Kryolagersystems soll nachfolgend anhand der Fig. 1 und 2 erläutert werden. In einem ersten Betriebszustand des Kryolagersystems gemäß Fig. 1 sind in den Schubläden 11 Proben enthalten, von denen Information wie die Art der Probe und ihrer Lokalisierung in den jeweiligen Probenträgern 10 und den Schubladen 11 sowie ihre Position innerhalb der Schublade 11 bekannt und abrufbar gespeichert sind.

Soll eine bestimmte Probe aus dem Kryolagersystem entnommen werden, werden die oben genannten Informationen zur Lokalisierung der Probe an die Steuerung 8 gesendet. Von dieser ergeht ein Steuerbefehl an den Antrieb 7 der Probenträgeraufnahme 4. Die Probenträgeraufnahme 4 wird um die Drehachse 9 gedreht, bis sich der die gewünschte Probe enthaltende Probenträger 10 unter dem ersten Zugang 1.1 befindet und diesem zugestellt ist. Durch die Zustellbewegung sind die ersten Kupplungselemente 13.1 des zugestellten Probenträgers 10 und die zweiten Kupplungselemente 13.2 des ersten Verschlusses 1.2 so gestellt, dass diese ineinander eingreifen ohne sich zu berühren.

Durch einen weiteren Steuerbefehl von der Steuerung 8 an den Kettenantrieb 15 wird durch diesen über die Kette 16 der erste Verschluss 1.2 vertikal nach oben gezogen. Dabei gelangen die ersten und zweiten Kupplungselemente 13.1, 13.2 miteinander in formschlüssigen Kontakt. Der Probenträger 10 wird von der Probenträgeraufnahme 4 abgehoben und durch den ersten Zugang 1.1 in das Zwischenlager 2 hinein transportiert, wie dies in Fig. 2 gezeigt ist. Das Zwischenlager 2 weist einen vertikale Temperaturverlauf auf, der mittels der Kühleinrichtung 17 so eingestellt ist, dass keine der Schubladen 11 des Probenträgers 10 in Bereiche mit Temperaturen wärmer als die Arbeitstemperatur gelangt.

Zur Vermeidung von Eisbildung und zur Gewährleistung eines schnellen Probenzugriffs, sind das Zwischenlager 2 und der Arbeitsraum 3 durchgängig gekühlt (Stand-by-Funktion).

Es ist in einer weiteren Ausführung des erfindungsgemäßen Kryolagersystem auch möglich, dass zuerst das Zwischenlager 2 auf eine bestimmte Temperatur, z. B. - 100°C, gekühlt wird. Der Arbeitsraum 3 wird durch ein hinsichtlich seiner Dauer und seines Grades (z. B. hälftige oder vollständige Wegnahme des zweiten Verschlusses 2.2) gesteuertes Öffnen des zweiten Zugangs 2.1, auf eine bestimmte Temperatur, z. B. zwischen -20° und -100°C, gekühlt. Das Öffnen des zweiten Verschlusses 2.2 kann, unterstützend zur Regelung der Kühleinrichtung 17, zur Temperaturreglung des Arbeitsraums 3 sowie zur Einstellung gewünschter vertikaler Temperaturverläufe in Zwischenlager 2 und Arbeitsraum 3 genutzt sein. Durch ein Öffnen des zweiten Zugangs 2.1 durch Wegnahme des zweiten Verschlusses 2.2 während des Kühlens wird ein schneller Zugriff zu den Proben ermöglicht sowie Kühlmedium und Zeit gespart.

In dem Zwischenlager 2 ist eine vertikale Position der Übergabeposition 26 definiert, zu der die Schublade 11 mit der Probe zugestellt wird.

Zu diesem Zeitpunkt ist der erste Zugang 1.1 geöffnet, während der zweite Zugang 2.1 noch geschlossen ist.

Nach Zustellung der Schublade 11 zur Übergabeposition 26 wird der zweite Zugang 2.1 geöffnet.

Mittels des Antriebs 18.1 wird die Transportplattform 18.2 durch den zweiten Zugang 2.1 hindurch in der Transportebene 18.3 unter die Schublade 11 mit der Probe verfahren. Anschließend wird der Probenträger 10 aufgrund eines Steuerbefehls der Steuerung 8 an den Kettenantrieb 15 um einen solchen Betrag abgesenkt, dass die Schublade 11 mit der Transportplattform 18.2 in Kontakt gelangt und ausgehoben wird.

In einer alternativen Ausführung kann die Transportplattform 18.2 um einen bestimmten Betrag angehoben werden.

Durch Wirkung des Antriebs 18.1 der Transportvorrichtung 18 wird die Transportplattform 18.2 mit der Schublade 11 aus dem Probenträger 10 entnommen und durch den zweiten Zugang 2.1 in den Arbeitsraum 3 transportiert.

Durch den zweiten Zugang 2.1 tritt tiefkaltes Kühlmedium von dem Zwischenlager 2 in den Arbeitsraum 3 ein, wodurch dieser gekühlt ist. Durch die Dauer der Öffnung des zweiten Verschlusses 2.2 sowie der Regelung der in dem Zwischenlager 2 befindlichen Kühleinrichtung 17 ist die vertikale Temperaturschichtung in dem Arbeitsraum 3 einstellbar.

Nach dem Transport kann der zweite Zugang 2.1 wieder verschlossen werden. Es ist auch möglich, den Probenträger 10 in eine andere Übergabeposition 26 zu verfahren oder diesen wieder in den Kryoraum 1 abzusenken. Auch ist ein Transport eines anderen Probenträgers 10 in das Zwischenlager 2 möglich.

Die in den Arbeitsraum 3 transportierte Schublade 11 wird durch das Kontaktierungsmittel 19 an einem Speichermittel der Schublade 11 kontaktiert und die erfassten Daten mit den in einer Datenbank oder der Steuerung 8 hinterlegten Daten verglichen. Dadurch kann überprüft werden, ob die korrekte Schublade 11 entnommen wurde. Zudem kann die Entnahme der Schublade 11 und ggf. der Probe in der Datenbank dokumentiert werden. Ist auch die Probe mit einem Speichermittel versehen, wird auch dieses kontaktiert, wodurch die Richtigkeit der Position der Probe kontrolliert werden kann.

An die Arretiervorrichtung 20 wird ein mit flüssigen Stickstoff tiefkalt gefülltes Probengefäß 22 zugestellt und an dieser arretiert. Zugleich wird das Heizelement 21.3 des Dichtungselements 21 angeschalten, so dass die Dichtungsgummis 21.2 auf einer Temperatur wärmer als -40°C gehalten sind.

Der dritte Zugang 3.1 wird durch eine Bedienperson mittels der Handschuhe durch die Eingriffe 23 hindurch geöffnet und die Probe wird aus der Schublade 11 entnommen und durch den dritten Zugang 3.1 in das Probengefäß 22 eingebracht.

Anschließend wird der dritte Zugang 3.1 geschlossen und das Probengefäß 22 von der Arretiervorrichtung 20 gelöst.

Die Schublade 11 wird mittels der Transportplattform 18.2 durch den nun wieder geöffneten zweiten Verschluss 2.2 wieder in den Probenträger 10 eingeschoben und der Probenträger 10 angehoben, so dass die Schublade 11 wieder im Probenträger 10 aufgenommen ist. Die Transportplattform 18.2 wird in den Arbeitsraum 3 zurückbewegt. Der Probenträger 10 wird anschließend durch Absenken des ersten Verschlusses 1.2 in den Kryoraum 1 transportiert und auf der Probenträgeraufnahme 4 abgesetzt. Erster und zweiter Zugang 1.1, 2.1 sind wieder geschlossen.

Die erfindungsgemäße Kryolagersystem kann für die Aufnahme, Speicherung und Ausgabe von Proben, insbesondere biologischer Proben, z.B. in den Bereichen der medizinischen, pharmazeutischen und chemischen Forschung sowie im klinischen Bereich eingesetzt werden.

### Bezugszeichenliste

- 1: Kryoraum
- 1.1: erster Zugang
- 1.2: erster Verschluss
- 1.3: Kryoraumdecke
- 2: Zwischenlager
- 2.1: zweiter Zugang
- 2.2: zweiter Verschluss
- 3: Arbeitsraum
- 3.1: dritter Zugang
- 3.2: dritter Verschluss
- 4: Probenträgeraufnahme
- 4.1: Platte
- 4.2: Bockrolle
- 5: Welle
- 6: Lager
- 7: Antrieb (der Probenträgeraufnahme)
- 8: Steuerung
- 9: Drehachse
- 10: Probenträger
- 11: Schubladen
- 12: Wartungszugang
- 12.1: Verschluss (des Wartungszugangs)
- 13: Kupplung
- 13.1: erstes Kupplungselement
- 13.2: zweites Kupplungselement
- 14: Trennwand
- 15: Kettenantrieb
- 16: Kette
- 17: Kühleinrichtung
- 18: Transportvorrichtung
- 18.1: Antrieb
- 18.2: Transportplattform
- 18.3: Transportebene
- 19: Kontaktierungsmittel
- 20: Arretiervorrichtung
- 21: Dichtungselement
- 21.1: Halterung
- 21.2: Dichtungsgummi
- 21.3: Heizelement
- 22: Probengefäß
- 23: Eingriff
- 24: Sichtfenster
- 25: Zustellvorrichtung
- 26: Übergabeposition
- 27: Warmbereich
- a: vertikale Ausdehnung

## Patentansprüche

1. Kryolagersystem zur Aufnahme, Speicherung und Ausgabe von Proben umfassend einen thermisch isolierten Kryoraum mit darin befindlichen Probenträgern zur Aufnahme der Proben und einem verschließbaren ersten Zugang in einem oberen Deckenbereich des Kryoraums, über den der Kryoraum mit einem über dem Kryoraum angeordneten thermisch isolierten Zwischenlager in Verbindung steht; mit Mitteln zum Transport mindestens eines Probenträgers zwischen dem Kryoraum und dem Zwischenlager; mit einem seitlich an das Zwischenlager anschließenden Arbeitsraum, in dem Mittel zum Transport in mindestens einer horizontalen Richtung einzeln aus dem Probenträger entnehmbarer und wieder in diesen einsteckbarer Elemente eines im Zwischenlager befindlichen Probenträgers in den Arbeitsraum angeordnet sind und in dem eine Schleuse zum Transport von Proben zwischen einem Probentransportgefäß und dem Arbeitsraum vorhanden ist,
wobei
- in dem Kryoraum (1) eine Probenträgeraufnahme (4) zur Aufnahme mehrerer Probenträger (10) vorhanden ist;
- die Probenträgeraufnahme (4) beweglich gelagert und mit einem gesteuerten Antrieb (7) in Verbindung steht, so dass die einzelnen Probenträger (10) dem ersten Zugang (1.1) des Kryoraums (1) zustellbar sind;
- das Zwischenlager (2) und der Arbeitsraum (3) gegeneinander thermisch isoliert sind;
- das Zwischenlager (2) und der Arbeitsraum (3) miteinander über einen verschließbaren zweiten Zugang (2.1) in Verbindung stehen;
- der Boden des Arbeitsraums (3) mindestens über einen Bereich von unten und von außerhalb des Kryoraums (1) zugänglich ist und in dem Boden des Arbeitsraums (3) ein verschließbarer dritter Zugang (3.1) mit einem Dichtungselement (21) und einer Arretiervorrichtung (20) zur lösbaren Arretierung eines Probengefäßes (22) derart vorhanden sind, dass das Probengefäß (22) gasdicht an dem dritten Zugang (3.1) des Arbeitsraums (3) arretierbar ist und Proben zwischen einem arretierten Probengefäß (22) und dem Arbeitsraum (3) transportiert werden können.

2. Kryolagersystem nach Anspruch 1, **dadurch gekennzeichnet, dass** die entnehmbaren und wieder in den Probenträger (10) einsteckbaren Elemente der Probenträger (10) einzelne Schubladen (11) sind, welche jeweils eine Grundfläche eines Zylindersektors aufweisen.

3. Kryolagersystem nach Anspruch 2, **dadurch gekennzeichnet, dass** die Schubladen (11) eines in dem Zwischenlager (2) befindlichen Probenträgers (10) durch Positionieren des Probenträgers (10) einer Übergabeposition (26) zustellbar sind und die Mittel zum Transport der Schubladen (11) zwischen einem im Zwischenlager (2) befindlichen Probenträger (10) und dem Arbeitsraum (3) der Übergabeposition (26) zustellbar sind, wobei die Übergabeposition (26) so definiert ist, dass ein Transport von Schubladen (11) durch den zweiten Zugang (2.1) möglich ist.

4. Kryolagersystem nach einem der vorhergehenden Ansprüche, **dadurch gekennzeichnet, dass** der zweite Zugang (2.1) zwischen Zwischenlager (2) und Arbeitsraum (3) eine vertikale Ausdehnung (a) aufweist, über deren Bereich Schubladen (11) horizontal zwischen dem Zwischenlager (2) und dem Arbeitsraum (3) transportierbar sind und die vertikale Ausdehnung (a) des zweiten Zugangs (2.1) zu den jeweiligen Deckenbereichen des Zwischenlagers (2) und des Arbeitsraums (3) beabstandet endet.

5. Kryolagersystem nach Anspruch 4, **dadurch gekennzeichnet, dass** die Probenträgeraufnahme (4) um eine koaxial einer Drehachse (9) verlaufende Welle (5) angeordnet und mit dieser verbunden ist, wobei die Welle (5) an ihrem im Kryoraum (1) befindlichen Ende in einem Lager (6) um die Drehachse (9) drehbar gelagert ist und die Probenträgeraufnahme (4) mit ihrer Unterseite auf Bockrollen (4.2) abgestützt ist, welche aus Richtung des Bodens des Kryoraums (1) in Richtung der Unterseite der Probenträgeraufnahme (4) ragen.

6. Kryolagersystem nach einem der vorhergehenden Ansprüche, **dadurch gekennzeichnet, dass** die Dimensionierung des Zwischenlagers (2) die gleichzeitige Aufnahme immer nur eines Probenträgers (10) erlaubt.

7. Kryolagersystem nach Anspruch 6, **dadurch gekennzeichnet, dass** an dem ersten Zugang (1.1) ein erster Verschluss (1.2) vorhanden ist, der an seiner in den Kryoraum (1) weisenden Seite erste Kupplungselemente (13.1) aufweist und jeder Probenträger (10) zweite Kupplungselemente (13.2) aufweist, wobei durch die ersten Kupplungselemente (13.1) und die zweiten Kupplungselemente (13.2) eine Kupplung (13) zur lösbaren Verbindung des ersten Verschlusses (1.2) und einem, dem ersten Zugang (1.1) zugestellten, Probenträger (10) gebildet ist, mittels der je ein an dem ersten Verschluss (1.2) angekuppelter Probenträger (10) zwischen dem Kryoraum (1) und dem Zwischenlager (2) transportierbar ist.

8. Kryolagersystem nach Anspruch 7, **dadurch gekennzeichnet, dass** der erste Verschluss (1.2) über eine Kette (15) mit einem Kettenantrieb (16) verbunden ist.

9. Kryolagersystem nach einem der vorhergehenden Ansprüche, **dadurch gekennzeichnet, dass** die Mittel zum Transport der Schubladen (11) zwischen dem im Zwischenlager (2) befindlichen Probenträger (10) und dem Arbeitsraum (3) eine Transportvorrichtung (18) ist, die mittels eines Zugmittels angetrieben sind und durch die eine in den Arbeitsraum (3) transportierte Schublade (11) einer Zugriffsposition mit definierten Lagekoordinaten im Arbeitsraum (3) zustellbar ist.

10. Kryolagersystem nach Anspruch 9, **dadurch gekennzeichnet, dass** an jeder Schublade (11) Speichermittel zur Speicherung und Bereitstellung von Daten vorhanden sind.

11. Kryolagersystem nach Anspruch 10, **dadurch gekennzeichnet, dass** in dem Arbeitsraum (3) Kontaktierungsmittel (19) zur Kontaktierung der Speichermittel einer in den Arbeitsraum (3) transportierten Schublade (11) vorhanden sind, wobei durch die Kontaktierung Daten zwischen dem Kontaktierungsmittel (19) und dem Speichermittel austauschbar sind.

12. Kryolagersystem nach Anspruch 1, **dadurch gekennzeichnet, dass** das Dichtungselement (21) beheizbar ist.

13. Kryolagersystem nach einem der vorhergehenden Ansprüche, **dadurch gekennzeichnet, dass** in dem Arbeitsraum (3) mindestens ein Eingriff (23) zur Manipulation von Proben durch den dritten Zugang (3.1) und im Arbeitsraum (3) vorhanden ist.

14. Kryolagersystem nach einem der vorhergehenden Ansprüche, **dadurch gekennzeichnet, dass** in dem Arbeitsraum (3) eine gesteuert arbeitende Manipulationsvorrichtung zur Manipulation von Proben durch den dritten Zugang (3.1) und im Arbeitsraum (3) vorhanden ist.

## Claims

1. A cryogenic storage system for receiving, storing and dispensing samples comprising a thermally insulated cryogenic space with sample carriers arranged therein for receiving the samples and a sealable first access in an upper ceiling portion of the cryogenic space through which the cryogenic space communicates with a thermally insulated intermediate storage disposed above the cryogenic space; means for transporting at least one sample carrier between the cryogenic space and the intermediate storage; with a working space laterally adjoining the intermediate storage, in which means are arranged for transporting, in at least one horizontal direction, elements of a sample carrier located in the intermediate storage, which elements can be individually removed from the sample carrier and inserted into the sample carrier again, into the working space, and in which a lock for transporting samples between a sample transport vessel and the working space is provided, wherein
- a sample carrier receptacle (4) for receiving a plurality of sample carriers (10) is present in the cryogenic space (1);
- the sample carrier receptacle (4) is movably mounted and connected to a controlled drive (7) so that the individual sample carriers (10) can be advanced to the first access (1.1) of the cryogenic space (1);
- the intermediate storage (2) and the working space (3) are thermally insulated from one another;
- the intermediate storage (2) and the working space (3) are connected to one another via a closable second access (2.1);
- the floor of the working space (3) is accessible at least over a region from below and from outside the cryogenic space (1), and in the floor of the working space (3) there is a closable third access (3.1) having a sealing element (21) and a locking device (20) for releasably locking a sample vessel (22) in such a way that the sample vessel (22) can be locked in a gas-tight manner at the third access (3.1) of the working space (3) and samples can be transported between a locked sample vessel (22) and the working space (3).

2. The cryogenic storage system according to claim 1, **characterized in that** the removable elements of the sample carriers (10) which can be inserted again into the sample carrier (10) are individual drawers (11) which each have a base surface of a cylinder sector.

3. The cryogenic storage system according to claim 2, **characterized in that** the drawers (11) of a sample carrier (10) located in the intermediate storage (2) can be advanced to a transfer position (26) by positioning the sample carrier (10), and the means for transporting the drawers (11) between a sample carrier (10) located in the intermediate storage (2) and the working space (3) can be advanced to the transfer position (26), the transfer position (26) being defined so as to enable a transport of drawers (11) through the second access (2.1).

4. The cryogenic storage system according to any one of the preceding claims, **characterized in that** the second access (2.1) has a vertical extension (a) between the intermediate storage (2) and the working space (3), over the region of which drawers (11) can be transported horizontally between the intermediate storage (2) and the working space (3) and the vertical extension (a) of the second access (2.1) ends at a distance from the respective ceiling regions of the intermediate storage (2) and of the working space (3).

5. The cryogenic storage system according to claim 4, **characterized in that** the sample carrier receptacle (4) is arranged about a shaft (5) extending coaxially with respect to an axis of rotation (9) and is connected thereto, wherein the shaft (5) is mounted at its end located in the cryogenic space (1) in a bearing (6) so as to be rotatable about the axis of rotation (9) and the underside of the sample carrier receptacle (4) is mounted on fixed castors (4.2), which protrude from the direction of the bottom of the cryogenic space (1) in the direction of the underside of the sample carrier receptacle (4).

6. The cryogenic storage system according to any one of the preceding claims, **characterized in that** the dimensioning of the intermediate storage (2) permits the simultaneous reception of only one sample carrier (10) at a time.

7. The cryogenic storage system according to claim 6, **characterized in that** a first closure (1.2) is present at the first access (1.1), which has first coupling elements (13.1) on its side pointing into the cryogenic space (1) and each sample carrier (10) has second coupling elements (13.2), the first coupling elements (13.1) and the second coupling elements (13.2) forming a coupling (13) for releasably connecting the first closure (1.2) and a sample carrier (10) which is advanced to the first access (1.1) and by means of which coupling (13) a respective sample carrier (10) coupled to the first closure (1.2) can be transported between the cryogenic space (1) and the intermediate storage (2).

8. The cryogenic bearing system according to claim 7, **characterized in that** the first closure (1.2) is connected to a chain drive (16) via a chain (15).

9. The cryogenic storage system according to any one of the preceding claims, **characterized in that** the means for transporting the drawers (11) between the sample carrier (10) located in the intermediate storage (2) and the working space (3) is a transport device (18) driven by a traction device and by means of which a drawer (11) transported into the working space (3) can be advanced to an access position with defined position coordinates in the working space (3).

10. The cryogenic storage system according to claim 9, **characterized in that** storage means for storing and providing data are present on each drawer (11).

11. The cryogenic storage system according to claim 10, **characterized in that** contacting means (19) for contacting the storage means of a drawer (11) transported into the working space (3) are present in the working space (3), data being exchangeable between the contacting means (19) and the storage means by said contacting.

12. The cryogenic storage system according to claim 1, **characterized in that** the sealing element (21) is heatable.

13. The cryogenic storage system according to any one of the preceding claims, **characterized in that** at least one intervention opening (23) is present in the working space (3) for manipulating samples through the third access (3.1) and in the working space (3).

14. The cryogenic storage system according to any one of the preceding claims, **characterized in that** in the working space (3) there is a controlled manipulation device for manipulating samples through the third access (3.1) and in the working space (3).

## Revendications

1. Système de stockage cryogénique pour recevoir, stocker et distribuer des échantillons comprenant un espace cryogénique thermiquement isolé dans lequel se trouvent des portes-échantillon pour recevoir les échantillons et un premier accès verrouillable dans une partie supérieure du plafond de l'espace cryogénique par lequel l'espace cryogénique communique avec un stockage intermédiaire thermiquement isolé disposé au-dessus de l'espace cryogénique ; un moyen pour transporter au moins un porte-échantillon entre l'espace cryogénique et le stockage intermédiaire ; avec un espace de travail contigu latéralement au stockage intermédiaire, dans lequel espace de travail sont disposés des moyens pour transporter dans celui-ci, dans au moins une direction horizontale, des éléments d'un porte-échantillon situé dans le stockage intermédiaire, lesquels éléments peuvent être retirés individuellement du porte-échantillon et réinsérés dans celui-ci, et dans lequel est prévue un sas pour transporter des échantillons entre un récipient de transport d'échantillons et l'espace de travail, où
- un réceptacle de porte-échantillon (4) destiné à recevoir plusieurs portes-échantillon (10) est présent dans l'espace cryogénique (1) ;
- le réceptacle de porte-échantillon (4) est monté de manière mobile et relié à un entraînement commandé (7) de sorte que les différents portes-échantillon (10) peuvent être amenés au premier accès (1.1) de l'espace cryogénique (1) ;
- le stockage intermédiaire (2) et l'espace de travail (3) sont isolés thermiquement l'un de l'autre ;
- le stockage intermédiaire (2) et l'espace de travail (3) sont reliés entre eux par un deuxième accès (2.1) verrouillable ;
- le fond de l'espace de travail (3) est accessible au moins sur une zone par le dessous et par l'extérieur de l'espace cryogénique (1), et dans le fond de l'espace de travail (3) il y a un troisième accès (3.1) verrouillable, comportant un élément d'étanchéité (21) et un dispositif de verrouillage (20) pour verrouiller de manière amovible un récipient d'échantillon (22) de telle sorte que le récipient d'échantillon (22) peut être verrouillé de manière étanche au gaz au niveau du troisième accès (3.1) de l'espace de travail (3) et des échantillons peuvent être transportés entre un récipient d'échantillon verrouillé (22) et l'espace de travail (3).

2. Système de stockage cryogénique selon la revendication 1, **caractérisé en ce que** les éléments des portes-échantillon (10) qui peuvent être retirés du porte-échantillon (10) et réinsérés dans celui-ci sont des tiroirs individuels (11) ayant chacun une surface de base d'un secteur cylindrique.

3. Système de stockage cryogénique selon la revendication 2, **caractérisé en ce que** les tiroirs (11) d'un porte-échantillon (10) situé dans le stockage intermédiaire (2) peuvent être avancés vers une position de transfert (26) en positionnant le porte-échantillon (10), et les moyens pour transporter les tiroirs (11) entre un porte-échantillon (10) situé dans le stockage intermédiaire (2) et l'espace de travail (3) peuvent être avancés vers la position de transfert (26), la position de transfert (26) étant définie de telle sorte qu'un transport de tiroirs (11) à travers le deuxième accès (2.1) soit rendu possible.

4. Système de stockage cryogénique selon l'une quelconque des revendications précédentes, **caractérisé en ce que** le deuxième accès (2.1) présente une extension verticale (a) entre le stockage intermédiaire (2) et l'espace de travail (3), sur laquelle des tiroirs (11) peuvent être transportés horizontalement entre le stockage intermédiaire (2) et l'espace de travail (3) et l'extension verticale (a) du deuxième accès (2.1) se termine à une distance des zones de plafond respectives du stockage intermédiaire (2) et de l'espace de travail (3).

5. Système de stockage cryogénique selon la revendication 4, **caractérisé en ce que** le réceptacle de porte-échantillon (4) est disposé autour d'un arbre (5) s'étendant coaxialement à un axe de rotation (9) et y est relié, l'arbre (5) étant monté à son extrémité située dans l'espace cryogénique (1) dans un palier (6), de manière à pouvoir être tourné autour de l'axe de rotation (9), et le récepteur de porte-échantillon (4) étant monté sur des rouleaux fixes (4.2) avec sa face inférieure, qui dépassent du fond de l'espace cryogénique (1) en direction de la face inférieure du réceptacle de porte-échantillon (4).

6. Système de stockage cryogénique selon l'une quelconque des revendications précédentes, **caractérisé en ce que** le dimensionnement du stockage intermédiaire (2) permet la réception simultanée d'un seul porte échantillon (10) à la fois.

7. Système de stockage cryogénique selon la revendication 6, **caractérisé en ce qu'**une première fermeture (1.2) est présente au niveau du premier accès (1.1), qui présente des premiers éléments d'accouplement (13.1) sur son côté dirigé vers l'espace cryogénique (1), et chaque porte-échantillon (10) présente des deuxièmes éléments d'accouplement (13.2), les premiers éléments d'accouplement (13.1) et les deuxièmes éléments d'accouplement (13.2) formant un accouplement (13) pour relier de manière amovible la première fermeture (1.2) et un porte-échantillon (10) qui est amené au premier accès (1.1) et au moyen duquel accouplement un porte-échantillon (10) couplé à la première fermeture (1.2) peut être transporté entre l'espace cryogénique (1) et le stockage intermédiaire (2).

8. Système de stockage cryogénique selon la revendication 7, **caractérisé en ce que** la première fermeture (1.2) est reliée par une chaîne (15) à un entraînement à chaîne (16).

9. Système de stockage cryogénique selon l'une quelconque des revendications précédentes, **caractérisé en ce que** le moyen pour transporter les tiroirs (11) entre le porte-échantillon (10) situé dans le stockage intermédiaire (2) et l'espace de travail (3) est un dispositif de transport (18) qui est entraîné par un moyen de traction et par lequel un tiroir (11) transporté dans l'espace de travail (3) peut être amené à une position d'accès ayant des coordonnées définies dans l'espace de travail (3).

10. Système de stockage cryogénique selon la revendication 9, **caractérisé en ce que** des moyens de stockage pour stocker et fournir des données sont présents sur chaque tiroir (11).

11. Système de stockage cryogénique selon la revendication 10, **caractérisé en ce que** des moyens de contact (19) pour entrer en contact avec les moyens de stockage d'un tiroir (11) transporté dans l'espace de travail (3) sont présents dans l'espace de travail (3), ce contact permettant d'échanger des données entre les moyens de contact (19) et les moyens de stockage.

12. Système de stockage cryogénique selon la revendication 1, **caractérisé en ce que** l'élément d'étanchéité (21) est chauffable.

13. Système de stockage cryogénique selon l'une quelconque des revendications précédentes, **caractérisé en ce qu'**au moins une ouverture d'intervention (23) est présente dans l'espace de travail (3) pour manipuler des échantillons par le troisième accès (3.1) et dans l'espace de travail (3).

14. Système de stockage cryogénique selon l'une quelconque des revendications précédentes, **caractérisé en ce que** dans l'espace de travail (3) se trouve un dispositif de manipulation commandé pour manipuler des échantillons par le troisième accès (3.1) et dans l'espace de travail (3).
